**Europäisches Patentamt**

**European Patent Office**

**Office européen des brevets**

(19)

(11) Publication number: **0 176 638**
**B1**

(12)

# EUROPEAN PATENT SPECIFICATION

(45) Date of publication of patent specification: **23.08.89**

(21) Application number: **84306732.3**

(22) Date of filing: **03.10.84**

(51) Int. Cl.⁴: **G 01 N 33/543,**
**G 01 N 33/546,**
**G 01 N 33/548**

(54) **Artificial support material for immobilisation of biological protein and process for producing the same.**

(43) Date of publication of application:
**09.04.86 Bulletin 86/15**

(45) Publication of the grant of the patent:
**23.08.89 Bulletin 89/34**

(84) Designated Contracting States:
**CH DE FR GB IT LI NL**

(56) References cited:
**EP-A-0 106 495**
**WO-A-83/03426**
**FR-A-2 334 106**
**US-A-4 169 804**
**US-A-4 335 094**
**US-A-4 416 813**
**US-A-4 438 239**

**CHEMICAL ABSTRACTS, vol. 102, no. 9, 4th March 1985, page 285, no. 75281a, Columbus, Ohio, US; & JP - A - 59 195 161 (FUJIREBIO, INC.) 06-11-1984**

(73) Proprietor: **FUJIREBIO KABUSHIKI KAISHA also trading as FUJIREBIO INC.**
**6-7, Shimoochiai 4-chome Shinjuku-ku Tokyo 161 (JP)**

(72) Inventor: **Ikeda, Mikio**
**7-25-23, Sunagawa-cho**
**Tachikawa-shi Tokyo (JP)**
Inventor: **Sakamoto, Shiro**
**2460, Hino**
**Hino-shi Tokyo (JP)**
Inventor: **Suzuki, Kazumasa**
**3-24-1-201, Motobuto**
**Urawa-shi Saitama-ken (JP)**

(74) Representative: **Silverman, Warren et al**
**HASELTINE LAKE & CO. Hazlitt House 28 Southampton Buildings Chancery Lane London WC2A 1AT (GB)**

EP 0 176 638 B1

Courier Press, Leamington Spa, England.

## Description

This invention relates to an artificial support material for use in serological testing and to a process for producing the same. The support material of the present invention is useful as a carrier to immobilise bioligical proteins such as antigens, antibodies and enzymes. Immobilised antigens, antibodies and enzymes are used in assays involving antigen-anti-body reactions.

Artificial support materials in particulate form which comprise organic polymers as principal ingredients may be of a variety of types. One such material consists of particles comprising gelatin having an isoelectric point of 8 to 9, a water-soluble polysaccharide and an alkali metal polymetaphosphate, which particles are insolubilized with respect to water by cross-linking treatment with an aldehyde. Such particles are described in our European Patent Application No. 82301235.6 (0062968).

It is an object of the present invention to provide a modification of such artificial support material which will enable one to achieve control of agglutination time in an antigen-antibody reaction and easy recovery of the support material used therein.

According to one aspect of this invention there is provided an artificial support for use in serological testing which is in the form of particles comprising gelatin, a water-soluble polysaccharide and an alkali metal polymetaphosphate, which particles are insolubilised with respect to water by cross-linking treatment with an aldehyde, and which is characterised in that the particles additionally comprise a ferromagnetic substance present in an amount of from 0.00001 to 2% by weight and in that the gelatin constitutes from 5 to 30% by weight of the particles, the water-soluble polysaccharide constitutes from 1 to 5% by weight of the particles, the alkali metal polyphosphate is a sodium polymetaphosphate present in an amount of from 0.01 to 1% by weight of the particles and water constitutes from 62 to 94% by weight of the particles.

In a second aspect, this invention provides a process for the production of an artificial support material for use in serological testing which comprises forming an aqueous solution containing gelatin, a water-soluble polysaccharide and an alkali metal polymetaphosphate having a temperature higher than the gelation temperature of said gelatin, adjusting the pH of said solution to a value of from 2.5 to 6.0 to produce an suspension of gelatin-containing particles and contacting said particles with an aldehyde cross-linking agent to render the particles insoluble in water, in which process the aqueous solution additionally contains suspended therein a ferromagnetic substance in colloidal form and said particles contain the ferromagnetic substance in an amount of from 0.00001 to 2% by weight, the gelatin in an amount of from 5 to 30% by weight, the water-soluble polysaccharide in an amount of from 1 to 5% by weight and the alkali metal polymetaphosphate in an amount of from 0.01 to 1% by weight, with water constituting from 62 to 94% by weight of the particles.

The magnetic support material of this invention has advantages as shown below when it is used as a carrier for immobilisation of biological protein:

(1) Agglutination time in an antigen-antibody reaction can be controlled by a magnet.

(2) Magnetic particles can be much more easily separated or recovered from a suspension thereof, compared with conventional support materials not containing magnetic substances, simply by application of magnetic force.

The fundamental principle of using magnetic particulate support materials has previously been proposed by K. Widder et al. However, such support materials are produced by covering magnetic particles with human serum albumin and then subjecting the particles in turn, to emulsification and heat treatment (see K. Widder, G. Flouret and A. Senyei: J. Pharm. Sci. 68, 79 (1979)). Widder's process has the disadvantage that it is difficult to produce homogeneous magnetic particulate materials by this procedure.

In contrast, it has now been found that homogeneous magnetic partculate support materials can be obtained by mixing fine particles of a ferromagnetic substance with the materials used to produce a support material of the type described in the aforementioned European patent application.

The support material particles of the invention preferably have a particle size of 0.8—50 μm and are water-insoluble and hydrophilic, the gelatin of the particles having been cross-linked for this purpose using aldehyde to form links between amino groups thereof.

In the particle of this invention, the gelatin used preferably has a molecular weight of about 100,000. Moreover the gelatin preferably has an isoelectric point of pH 8.4 to 9.1. The most preferred water-soluble polysaccharide is gum arabic, although carboxymethyl cellulose, sodium alginate and agar are also suitable for use in this invention. The sodium polymetaphosphate preferably has the formula $(NaPO_3)_n$ wherein n is an integer of 3 to 6. Ferromagnetic substances which can be used include ferrites having the formula $MFe_2O_4$ wherein M represents a divalent metal, preferably $Fe^{++}$, $Mn^{++}$, $Cu^{++}$, $Ni^{++}$ or $Co^{++}$. When producing the support material of this invention, the ferrites are preferably in the form of an aqueous suspension containing colloidal ferrites in an amount of about 30% w/v.

In a first step in producing a support material of this invention, an aqueous suspension colloid of a ferrite needs to be produced. An aqueous suspension colloid of a ferrite, for example $MnFe_2O_4$, can be prepared, for example, by the steps of: mixing 100 ml a 1 M $MnCl_2$ solution and 100 ml of a 2 M $FeCl_3$ solution, adding a 6 N NaOH solution dropwise to the mixture until the solution has a pH of 11, heating the mixture at 100°C for 30 minutes, washing the precipitate which has formed with hot water and dispersing the precipitate in about 100 ml of an aqueous solution (about 0.1% w/v) of a surfactant such as sodium oleate.

Aqueous suspension colloids of other ferrites, e.g. $CuFe_2O_4$, $NiFe_2O_4$ and $CoFe_2O_4$, can be prepared by repeating the procedure of preparing the aqueous suspension of $MnFe_2O_4$ as shown above, but using $CuCl_2$, $NiCl_2$ and $CoCl_2$ instead of $MnCl_2$. It will generally be necessary the reaction mixture to be heated at higher temperatures than 100°C in an oil bath or heated an autoclave.

When an aqueous suspension colloid of magnetite ($Fe_3O_4$), is to be used as the ferrite, it can be prepared by the steps of: mixing 100ml of a 1 M $FeCl_2$ solution and 100 ml of a 2 M $FeCl_3$ solution, adding 200 ml of a 6 N NaOH solution to the mixture to produce a precipitate, washing the precipitate with hot water and dispersing the precipitate in 100 ml of an aqueous solution (about 0.1% w/v) of a surfactant such as sodium oleate.

An aqueous suspension colloid of the chosen ferrites is preferably prepared which contains colloidal ferrite in an amount of about 30% w/v.

The artificial support material of this invention may then be produced as follows:

0.01 to 2 g of gelatin, 0.01 to 2 g of water soluble polysaccharide, 0.001 to 2 g of alkali metal polymetaphosphate and 0.01 to 5 g of colloidal ferrite (about 36 µl to 18 ml of aqueous suspension colloid of ferrite) are added to 100 ml of an aqueous solution containing about 0.1% (w/v) anionic or nonionic surfactant to produce a colloidal solution, and after the pH of the colloidal solution has been adjusted to 2.5 to 6.0 with an acid such as acetic acid, the gelatin is cross-linked with an aldehyde, for example glutaraldehyde or formaldehyde, at a temperature of from 5°C to 35°C with stirring.

An anionic surfactant which is used may be an alkylsulphosuccinate, alkylsulphomaleate, alkylsulphuric acid ester or polyoxyethylene alkyl ether sulphuric acid ester. A nonionic surfactant which is used may be a polyoxyethylene fatty acid ester, polyoxyethylene alkyl ether, polyoxyethylene alkyl phenyl ether or polyethylene glycol fatty acid ester. The alkyl group for both groups or surfactants has from 6 to 25 carbon atoms.

The present invention is further illustrated by the following examples.

### Example 1

One gram of gelatin having an isoelectric point of pH 9 was dissolved in 25 ml of water at 40°C and to this solution was added a 10% by wt. NaOH solution to adjust the pH to 9.0. A solution of 1 g of gum arabic in 20 ml of water at 40°C was then added and the mixture obtained was poured into 150 ml of a 30% v/v aqueous ethanol solution, and after stirring, there was added to this mixture, in turn, 0.8 ml of a 10% by wt. sodium hexametaphosphate solution, 1 ml of a 1% by wt. aqueous solution of an alkylsulphomaleate (trade name: Demol Ep, manufactured by Kao Soap Co.) and 60 µl of a suspension colloid of $Fe_3O_4$ prepared as described above, and the mixture obtained was stirred to achieve homogeneity. Subsequently, a 10% by wt. acetic acid solution was added dropwise to the mixture while it was kept at 40°C and the pH of the mixture was adjusted to 4.8 to form a particulate suspension. The particulate suspension was cooled to 5°C in an ice bath, and 0.65 g of glutaraldehyde was added. After stirring, the suspension was allowed to stand at 5°C overnight. Then, the suspension was centrifuged at 2000 rpm for 10 minutes, and the resulting particles were collected. The particles were suspended in a 0.005% by wt. Demol Ep solution, and the suspension was centrifuged again. This washing procedure was repeated three times, and the particles were suspended in 50 ml of a 4% v/v formaldehyde solution, and this suspension was allowed to stand at 5°C for one week.

By working in this manner, a suspension of magnetic particles comprising about 20% by wt. gelatin, about 2% by wt. gum arabic, about 0.1% by wt. $(NaPO_3)_6$, about 0.001% by wt. of $Fe_3O_4$ and about 78% by wt. of water, the particles having a particle size of about 3 µm, was obtained.

### Example 2

The procedure of Example 1 was repeated except that sodium trimetaphosphate and 600 µl of a suspension colloid of $MnFe_2O_4$ prepared by the above indicated procedure were used instead of sodium hexametaphosphate and 60 µl of the suspension colloid of $Fe_3O_4$.

As a result of operating in this manner a suspension of magnetic particles containing about 20% by wt. of gelatin, about 2% by wt. of gum arabic, about 0.1% by wt. of $(NaPO_3)_3$, about 0.005% by wt. of $MnFe_2O_4$ and about 78% by wt. of water, and having particle size of about 3.5 µm was obtained.

Magnetic particles in powder form can be obtained by separating out from suspension the magnetic particles and lyophilizing the wet magnetic particles.

In Examples 1 and 2, suspensions of magnetic particles containing 0.001% and 0.005% by wt. respectively of ferromagnetic substance and having particle sizes of about 3 µm and about 3.5 µm respectively were obtained. It is to be understood, however, that the composition of the magnetic particles constituting the support material of this invention can be changed by changing the amount of the ingredients to be used in the production thereof. Furthermore, the particle size of the magnetic particles can be varied by producing the magnetic particles under different conditions of temperature and solution pH or employing different stirring conditions.

A support material embodying this invention is suitable for use in the same type of perological tests as the support material of European Patent Application No. 82301235.6 (EP—A—0062968) to which reference is directed, but with the aforementioned added advantages of allowing control of agglutination time and easy recovery of the support material made possible by the presence of the ferrite, coupled with homogeneity of the particulate material.

**Claims**

1. An artificial support material for use in serological testing which is in the form of particles comprising gelatin, a water-soluble polysaccharide and an alkali metal polymetaphosphate, which particles are insolubilised with respect to water by cross-linking treatment with an aldehyde, characterised in that the particles additionally comprise a ferromagnetic substance present in an amount of from 0.00001 to 2% by weight and in that the gelatin constitutes from 5 to 30% by weight of the particles, the water-soluble polysaccharide constitutes from 1 to 5% by weight of the particles, the alkali metal polyphosphate is a sodium polymetaphosphate present in an amount of from 0.01 to 1% by weight of the particles and water constitutes from 62 to 94% by weight of the particles.

2. An artificial support material as claimed in claim 1, wherein said gelatin has an isoelectric point of from pH 8.4 to 9.1.

3. An artificial support material as claimed in claim 1 or 2, wherein the water-soluble polysaccharide is selected from gum arabic, carboxylmethyl cellulose, sodium alginate and agar.

4. An artificial support material as claimed in any preceding claim, wherein said alkali metal polymetaphosphate has the formula $(NaPO_3)_n$ wherein n is an integer of 3 to 6.

5. An artificial support material as claimed in any preceding claim, wherein said ferromagnetic substances is a ferrite having the formula $MFe_2O_4$ wherein M is a divalent metal selected from $Fe^{++}$, $Mn^{++}$, $Cu^{++}$, $Ni^{++}$ or $Co^{++}$.

6. A process for the production of an artificial support material for use in serological testing which comprises forming an aqueous solution containing gelatin, a water-soluble polysaccharide and an alkali metal polymetaphosphate having a temperature higher than the gelation temperature of said gelatin, adjusting the pH of said solution to a value of from 2.5 to 6.0 to produce an suspension of gelatin-containing particles and contacting said particles with an aldehyde cross-linking agent to render the particles insoluble in water, characterised in that said aqueous solution additionally contains suspended therein a ferromagnetic substance in colloidal form and said particles contain the ferromagnetic substance in an amount of from 0.00001 to 2% by weight, the gelatin in an amount of from 5 to 30% by weight, the water-soluble polysaccharide in an amount of from 1 to 5% by weight and the alkali metal polymetaphosphate in an amount of from 0.01 to 1% by weight, with water constituting from 62 to 94% by weight of the particles.

7. The process of claim 6, wherein said gelatin has an isoelectric point of from pH 8.4 to 9.1.

8. The process of claim 6 or 7, wherein the concentration of gelatin in said solution is from 0.01% to 2% by weight.

9. The process of claim 6, 7 or 8, wherein the concentration of water-soluble polysaccharide in said solution is from 0.01 to 2% by weight.

10. The process of any one of claims 6 to 9, wherein said water-soluble polysacchride is selected from gum arabic, carboxylmethyl cellulose, sodium alginate and agar.

11. The process of any one of claims 6 to 10, wherein said alkali metal phosphate is sodium polymetaphosphate present in said solution in a concentration of from 0.001% to 2% by weight.

12. The process of claim 11, wherein said sodium polymetaphosphate has the formula $(NaPO_3)_n$, wherein n is an integer of from 3 to 6.

13. The process of any one of claims 6 to 12, wherein said ferromagnetic substance is suspended in said solution in an amount of from 0.01% to 5% by weight.

14. The process of any one of claims 6 to 13, wherein said ferromagnetic substance is a ferrite having the formula $MFe_2O_4$, wherein M is a divalent metal selected from $Fe^{++}$, $Mn^{++}$, $Cu^{++}$, $Ni^{++}$ or $Co^{++}$.

15. The process of any one of claims 6 to 14, wherein said aldehyde is selected from glutaraldehyde and formaldehyde.

**Patentansprüche**

1. Künstliches Trägermaterial für serologische Tests in Form von Teilchen, die Gelatine, eine wasserlösliches Polysaccharid und eine Alkalimetall-Polymetaphoshat enthalten und durch Vernetzung mit einem Aldehyd wasserunlöslich gemacht wurden, dadurch gekennzeichnet, daß die Teilchen weiterhin eine ferromagnetische Substanz in einer Menge von 0,00001 bis 2 Gew.-% enthalten, die Gelatine 5 bis 30 Gew.-% und das wasserlösliche Polysaccharid 1 bis 5 Gew.-% der Teilchen ausmachen, das Alkalimetall-Polymetaphosphat ein Natrium-Polymetaphosphat ist und in einer Menge von 0.01 bis 1 Gew.-% in den Teilchen enthalten ist und Wasser 62 bis 94 Gew.-% der Teilchen ausmacht.

2. Künstliches Trägermaterial nach Anspruch 1, worin die Gelatine bei pH 8,4 bis 9,1 einen isoelektrischen Punkt hat.

3. Künstliches Trägermaterial nach Anspruch 1 oder 2, worin das wasserlösliche Polysaccharid aus der Reihe Gummiarabicum, Carboxymethylcellulose, Natrium-alginat und Agar ausgewählt ist.

4. Künstliches Trägermaterial nach einem der vorhergehenden Ansprüche, worin das Alkalimetall-Polymethaphosphat die Formel $(NaPO_3)_n$ hat, worin n eine ganze Zahl von 3 bis 6 ist.

5. Künstliches Trägermaterial nach einem der vorhergerhenden Ansprüche, worin die ferromagnetische Substanz ein Ferrit mit der Formel $MFe_2O_4$ ist, in der M ein zweiwertiges Metall aus der Reihe $Fe^{++}$, $Mn^{++}$, $Cu^{++}$, $Ni^{++}$ oder $Co^{++}$ ist.

6. Verfahren zur Herstellung eines künstlichen Trägermaterials für serologische Tests, wobei man eine Gelatine, in Wasser lösliches Polysaccharid und ein Alkalimetall-Polymethaphosphat enthaltende wässrige Lösung mit einer Temperatur, die höher ist als die Geliertemperatur der Gelatine, herstellt, den pH der Lösung auf einen Wert von 2,5 bis 6,0 einstellt, um eine Suspension von Gelatine-haltigen Teilchen zu erhalten, und diese Teilchen mit einem Aldehyd-Vernetzungsmittel zusammenbringt, um die Teilchen wasserunlöslich zu machen, dadurch gekennzeichnet, daß die wässrige Lösung weiterhin eine darin suspendierte ferromagnetische Substanz in kolloidaler Form und die Teilchen diese ferrognetische Substanz in einer Menge von 0,00001 bis 2 Gew.-%, die Gelatine in einer Menge von 5 bis 30 Gew.-%, das wasserlösliche Polysaccharid in einer Menge von 1 bis 5 Gew.-% und das Alkalimetall-Polymethaphosphat in einer Menge von 0,01 bis 1 Gew.-% enthalten, wobei Wasser 62 bis 94 Gew-% der Teilchen ausmacht.

7. Verfahren nach Anspruch 6, worin die Gelatine bei pH 8,4 bis 9,1 einen isoelektrischen Punkt hat.

8. Verfahren nach Anspruch 6 oder 7, worin die Gelatine-konzentration in der Lösung 0,01 bis 2 Gew.-% beträgt.

9. Verfahren nach Anspruch 6, 7 oder 8, worin die Konzentration des wasserlöslichen Polysaccharids in der Lösung 0,01 bis 2 Gew.-% beträgt.

10. Verfahren nach einem der Ansprüche 6 bis 9, worin das wasserlösliche Polysaccharid aus der Reihe Gummiarabicum, Crboxymethylcellulose, Natriumalginat und Agar ausgewählt wird.

11. Verfahren nach einem der Ansprüche 6 bis 10, worin das Alkalimittel-Polymethaphosphat Natriumpolymethaphosphat ist und in der Lösung in einer Konzentration von 0.001 bis 2 Gew.-% vorliegt.

12. Verfahren nach Anspruch 11, worin das Natriumpolymethaphosphat die Formel $(NaPO_3)_n$ hat, in der n eine ganze Zahl von 3 bis 6 bedeutet.

13. Verfahren nach einem der Ansprüche 6 bis 12, worin die ferromagnetische Substanz in der Lösung in einer Menge von 0.01 bis 5 Gew.-% suspendiert wird.

14. Verfahren nach einem der Ansprüche 6 bis 13, worin die ferromagnetische Substanz ein Ferrit mit der Formel $MFe_2O_4$ ist, in der M ein zweiwertiges Metall ist und aus der Reihe $Fe^{++}$, $Mn^{++}$, $Cu^{++}$, $Ni^{++}$ oder $Co^{++}$ ausgewählt wird.

15. Verfahren nach einem der Ansprüche 6 bis 14, worin das Aldehyd unter Glutaraldehyd und Formaldehyd ausgewählt wird.

## Revendications

1. Matériau de support artificiel, à utiliser dans des tests sérologiques, qui est sous la forme de particules comprenant de la gélatine, un polysaccharide soluble dans l'eau et un polymétaphosphate de métal alcalin, ces particules étant rendues insolubles dans l'eau par un traitement de réticulation avec un aldéhyde, caractérisé en ce qu'en plus, les particules contiennent une substance ferromagnétique présente en un quantité comprise entre 0,00001% et 2% en poids, et en ce que la gélatine représente de 5% à 30% en poids des particules, le polysaccharide soluble dans l'eau représente de 1% à 5% en poids des particules, le polyphosphate de métal alcalin est un polymétaphosphate de sodium présent en une quantité de 0.01% à 1% du poids des particules et l'eau représente de 62 à 94% en poids des particules.

2. Matériau de support artificiel selon la revendication 1, dans lequel ladite gélatine a un point isoélectrique situé entre pH 8,4 et pH 9,1.

3. Matériau de support artificiel selon la revendication 1 ou 2, dans lequel le polysaccharide soluble dans l'eau est choisi parmi la gamme arabique, la carboxyméthylcellulose, l'alginate de sodium et l'agar-agar.

4. Matériau de support artificiel selon l'une quelconque des revendications précédentes, dans lequel ledit polymétaphosphate de métal alcalin a la formule $(NaPO_3)_n$, dans laquelle n est un nombre entier de 3 à 6.

5. Matériau de support artificiel selon l'une quelconque des revendications précédentes, dans lequel ladite substance ferromagnétique est un ferrite ayant la formule $MFe_2O_4$, dans laquelle M est un métal divalent choisi parmi $Fe^{++}$, $Mn^{++}$, $Cu^{++}$, $Ni^{++}$ ou $Co^{++}$.

6. Procédé de préparation d'un matériau de support artificiel à utiliser dans des tests sérologiques, qui comprend la préparation d'un solution aqueuse contentant de la gélatine, un polysaccharide soluble dans l'eau et un polymétaphosphate de métal alcalin, à une température supérieur à la température de gélification de ladite gélatine, l'adjustement du pH de ladite solution à une valeur comprise entre 2,5 et 6,0, pour produire une suspension de particules contenant de la gélatine et la mise en contact desdites particules avec un agent de réticulation de type aldéhyde pour rendre les particules insolubles dans l'eau, caractérisé en ce que ladite solution aqueuse contient en outre en suspension, une substance ferromagnétique sous forme colloïdale, et en ce que lesdites particules contiennent la substance ferromagnétique en une quantité comprise entre 0,00001% et 2% en poids, le gélatine en une quantité de 5 à 30% en poids, le polysaccharide soluble dans l'eau en une quantité de 1 à 5% en poids, et le polymétaphosphate de métal alcalin en une quantité de 0.01% à 1% en poids, de l'eau constituant 62% à 94% en poids des particules.

7. Procédé de la revendication 6, dans lequel ladite gélatine a un point isoélectrique situé entre pH 8,4 et 9,1.

8. Procédé de la revendication 6 ou 7, dans lequel la concentration de gélatine dans ladite solution est comprise entre 0.01% et 2% en poids.

9. Procédé de la revendication 6, 7 ou 8, dans lequel la concentration du polysaccharide soluble dans l'eau dans ladite solution est comprise entre 0.01 et 2% en poids.

10. Procédé selon l'une quelconque des revendications 6 à 9, dans lequel ledit polysaccharide soluble dans l'eau est choisi parmi la gamme arabique, la carboxyméthylcellulose, l'alginate de sodium et l'agar-agar.

11. Procédé selon l'une quelconque des revendications 6 à 10, dans lequel ledit phosphate de métal alcalin est du polymétaphosphate de sodium, présent dans ladite solution à une concentration de 0,001% à 2% en poids.

12. Procédé selon la revendication 11, dans lequel ledit polymétaphosphate de sodium a la formule $(NaPo_3)_n$ dans laquelle n est un nombre entier de 3 à 6.

13. Procédé selon l'une quelconque des revendications 6 à 12, dans lequel ladite substance ferromagnétique est en suspension dans ladite solution en une quantité comprise entre 0.01% et 5% en poids.

14. Procédé selon l'une quelconque des revendications 6 à 13, dans laquelle ladite substance ferromagnétique est un ferrite ayant la formule $MFe_2O_4$, dans laquelle M est un métal divalent choisi parmi $Fe^{++}$, $Mn^{++}$, $Cu^{++}$, $Ni^{++}$ ou $Co^{++}$.

15. Procédé selon l'une quelconque des revendications 6 à 14, dans lequel ledit aldéhyde est choisi parmi le glutaraldéhyde et le formaldéhyde.